# EUROPEAN PATENT APPLICATION

(11) **EP 4 497 433 A1**
(43) Date of publication of application: **29.01.2025**
(21) Application number: 23187252.4
(22) Date of filing: 24.07.2023
(51) Int. Cl.: A61K 9/16, A61K 9/51, A61K 47/59, A61K 9/00, A61K 31/7088

(54) **NANO-IN-MICRO ENCAPSULATED SIRNA DRY POWDER AND PHARMACEUTICAL PRODUCT CONTAINING SAID DRY POWDER**

(71) Applicant: Ludwig-Maximilians-Universität München, 80539 München (DE)
(72) Inventor: Merkel, Olivia, 82234 Weißling (DE); Winkeljann, benjamin, 81371 München (DE); Jürgens, David Christian, 80538 München (DE); Deßloch Leonie, 80331 München (DE)
(74) Representative: Patent- und Rechtsanwälte Behrmann Wagner PartG mbB

(57) **Abstract**

The invention relates to Nano-in-micro (NIM) encapsulated bioactive siRNA dry powder comprising lipid nanoparticles, the lipid nanoparticles comprising from at least an ionizable cationic lipid, a helper lipid, a stealth lipid, and siRNA, wherein the lipid nanoparticles are encapsulated in sugar and/or sugar alcohol, wherein the mass ratio of siRNA to a sugar and/or sugar alcohol is more than 0.10%, especially between 0,90% and 1,30%. The invention also relates to a respective pharmaceutical product containing the dry powder in a sealed volume.

## Description

The current invention relates a Nano-in-Micro (NIM) encapsulated siRNA dry powder according to claim 1. The invention further relates to pharmaceutical product, in particular for the use with dry powder inhaler devices, comprising said dry powder in at least one sealed volume of a packaging according to claim 9.

Application of drugs directly to their site of action is the optimal way to reduce doses and side effects. For lung diseases such as asthma, pulmonary delivery is therefore favored. With a relatively low enzyme activity and a slow surface clearance, enzymatically prone substances are perfect candidates for this administration route. In addition, dry powder inhalers enable the delivery of drugs with a high shelf life and also provide an easy to use tool for patients along with high compliance. Despite several available treatments for lung diseases, nucleic acid therapy is a promising new tool to address uncontrollable disease variants such as severe, uncontrolled asthma, but also particularly viruses for which no antiviral compounds are available. Knowing the genome of the virus is sufficient to develop nucleic acid-based therapeutics that can inhibit viral replication. Small interfering RNA (siRNA) can silence the translation of messenger RNA into pathologically upregulated proteins and diminish disease symptoms. However, siRNA therapeutics face several challenges associated with the delivery into cells and enzymatic stability. To address these issues, nanoparticles are preferred to protect and encapsulate siRNA.

### State of the Art

In WO 2022/079105 A1 a method is disclosed how nano-in-micro encapsulated siRNA dry powder can be produced by spray drying from an aqueous suspension, leaving the siRNA intact, i.e. bioactive to a very large extent in the produced dry powder. This is a major advancement in the respective effort to provide for clinical doses of such gene therapy and the respective pharmaceuticals. However, in the state of the art the amount (mass ratio) of siRNA to sugar and in the produced dry powder is still relatively low. The prior art gives the mass ratio of siRNA to sugar and/or sugar alcohol to be preferably between 0,001% and 0,02 in order to exhibit the high yield or high percentage of intact/bioactive siRNA in the dry powder. With this amount or concentration of siRNA a treatment of a patient via inhaling the dry powder would require large amounts of repeated doses inhalations. Even if the inhalation of such a large amount of dry powder was possible for a patient, such a treatment or therapy would significantly reduce the patient's compliance.

However, increasing the amount or mass ratio of siRNA in a respective dry powder provides several problems, which need to be overcome. First, that the shielding effect of the polymers or lipid nano particles shielding the siRNA from environmental influences, especially heat, is likely to decrease if the mass ratio of the siRNA was increased. This makes the siRNA more prone to degrading influences in the production of the dry powder. As a result, the amount of bioactive siRNA could be reduced which in turn might not lead to a sufficient net increase of bioactive siRNA in the dry powder, Second, the increase of siRNA and lipid could lead to even stronger hygroscopic behaviour of the dry powder. This would adversely effect the shelf life of the dry powder and the delivery to the site of action, especially if this required passing through a humid and warm environment, such as an oropharynx of a patient, before reaching the site of action, i.e. the lung. Additionally, strong hygroscopic properties would make it increasingly difficult to dry the powder to a level that is acceptable. This might result in the need of higher temperatures during spry drying, which again might cause harm to or destroy the functionality of the siRNA. In addition, a higher mass ratio of lipid nano particles and siRNA provided in an aqueous suspension for spray drying would make the chemical/physical combination of the lipid nano particles with water soluble excipients, such as sugar and/or sugar alcohol more difficult causing problems with the micro encapsulation of the siRNA loaded lipid nano particles.

Accordingly, it is the aim of the present invention to propose a micro-in-nano encapsulated siRNA dry powder that enables a clinical use or use as a pharmaceutical dosage.

### Invention

This problem is solved by the Nano-in-micro (NIM) encapsulated bioactive siRNA dry powder according to claim 1. Advantageous embodiments of the invention are presented in the following description, in the drawings as well as in the dependent claims. Features hereinafter described or claims as device or product features shall also be considered to be disclosed and claimable as method features, and vice versa.

The Nano-in-micro (NIM) encapsulated bioactive siRNA dry powder according to the invention comprise lipid nanoparticles, the lipid nanoparticles comprising from at least an ionizable cationic lipid, a helper lipid, a stealth lipid, and siRNA, wherein the lipid nanoparticles are encapsulated in sugar and/or sugar alcohol, wherein the mass ratio of siRNA to a sugar and/or sugar alcohol is more than 0.10%, especially between 0,90% and 1,30%.

It was surprisingly found that the nano-in-micro encapsulated siRNA could be produced, especially by a spray drying method as disclosed in WO 2022/079105 A1 with a mass ratio of siRNA to a sugar and/or sugar alcohol that is high enough to enable a clinical or pharmaceutical application in humans.

The disclosure of WO 2022/079105 A1 is incorporated into this disclosure by reference with respect to the process/method of spry drying and the analysis of bioactivity of the siRNA in the dry powder.

It was proven possible to achieve this result without losing the shielding effect on the siRNA, thereby again producing a high yield of bioactive/intact spray dried siRNA. In addition, it was established that the dry powder could be produced as a dry powder without too much or too strong hygroscopic properties.

A difference compared to the spray drying process according the disclosure of WO 2022/079105 A1 was that prior to adding the lipid nano particles with the encapsulated siRNA to the water soluble excipients, preferably a 10% lactose solution in purified water, the concentration of lipid nano particles was raised. The concentration was determined by standard procedure and was necessary since the lipid nano particles can be produced only with a limited concentration. In a preferred variation of the known method, the concentration was raised to 60mM to 70mM from the concentration level of about 16mM of the direct preparation.

Surprisingly, the concentrated lipid nanoparticles could be combined with the water-soluble excipient without significant dispersion problems and could be spray dried without significant negative impact on the state or activity of the spray dried siRNA.

The used Ionizable Cationic Lipids can comprise or consist of lipids that can carry a positive charge under certain pH conditions. Their role includes facilitating nucleic acid encapsulation in lipid nano particles (LNPs), mediating endosomal membrane disruption for nucleic acid release, and possibly supporting endosomal uptake. Examples include lipids like 1,2-dioleoyl-3-dimethylammonium-propane (DODAP), DLin-MC3-DMA, being a key component in the formulation of LNPs used in the Pfizer-BioNTech and Moderna COVID-19 vaccines, and other similar ionizable lipids.

The used stealth lipids, often pegylated lipids or their alternatives, are added to LNPs to improve their stability, prolong circulation time in the body, and help evade the immune system. Examples of stealth lipids include PEG-lipids (e.g., DSPE-PEG2000), Polyvinylpyrrolidone (PVP) lipids, Polyvinyl alcohol (PVA) lipids, Poloxamer lipids, and Polysarcosine lipids. These examples all serve the purpose of providing a stealth characteristic to the LNPs, which enhances their stability and improves biodistribution.

The helper lipids support the stability of LNPs during storage and circulation. They can include a range of lipids such as sterols, phospholipids, and glycerolipids. Examples include cholesterol (a common sterol), DOPE (Dioleoylphosphatidylethanolamine, a type of phospholipid), and DOPC (Dioleoylphosphatidylcholine, another type of phospholipid). These lipids are typically non-cationic and help to improve the fluidity and flexibility of LNPs, as well as overall stability. In a preferred embodiment of the invention two helper lipids are used. When using two helper lipids it is especially advantageous that one of the helper lipids is cholesterol.

The choice of the respective set of lipids is largely dependent on the specific formulation and desired properties of the LNPs. Different applications may require adjustments in the types and ratios of these components.

According to a preferred embodiment of the dry powder, at least 75%, preferably at least 85% of the siRNA is bioactive in the dry powder state and/or after re-dispersion. The bioactivity of the spray-dried siRNA can be assessed as disclosed in WO 2022/079105 A1. In a preferred embodiment, the siRNA in the dry powder is structurally fully intact. In other words, the siRNA within the dry powder is free of degradation products. The high percentage of bioactive siRNA is especially important for the high mass ratio of siRNA of the dry powder. Because even if it was technologically possible to spray dry a powder with a high initial amount or ratio of siRNA the bioactivity after the spray drying determines whether the powder can be practically or conveniently used, for example as a pharmaceutical dosage for a dry powder inhaler (DPI).

According to another advantageous embodiment of the invention, the residual moisture of the dry powder is less than 5%, in particular less than 3%. This leads to a longer shelf life of the resulting dry powder. Preferably, an additional drying process can reach the residual moisture after the initial spray drying.

In accordance with an advantageous variation of the invention, the lipid mass ratio of the dry powder is above 8%, preferably above 10%. This is advantageous because high lipid mass ratio supports a high mass ratio of siRNA. Surprisingly even with such a high amount or mass ratio of lipids in the dry powder the hygroscopic properties of the dry powder were still within the acceptable range to be used as a dry powder for a dry inhaler.

According to a further embodiment of the invention, the sugar comprises or consists of lactose. It is believed that lactose used as sugar has a positive effect on the resulting dry powder. The respective mechanisms are, however, not yet fully understood or proven. Other excipients might as well be suitable for producing the dry powder.

In a further embodiment of the invention, the lipid nano particles are according to an Onpattro^{®}-formulation or patisiran formulation. The use of Onpattro^{®} being an already clinically approved substance and the respective Onpattro^{®}-formulation being readily available, makes the use of Onpattro^{®} and derivatives therefrom good starting points for the LNP as they are expected to receive clinical approval more conveniently. The respective lipid nano particles are disclosed in US 8,158,601 B2, which is hereby included by reference into the current disclosure. The respective formulation is documented by the EMA under the product name EU/3/11/857 and was given the non-proprietary name "PATISIRAN".

In another preferred embodiment, the dry powder is used as a pharmaceutical dosage form, in particular for pulmonary delivery. In this respect, it is especially advantageous that the dry powder, when produced by a spray drying process, such as disclosed in WO 2022/079105 A1, results in a particle size and size distribution which is suitable for pulmonary delivery. This allows for a direct use of the powder without further processing, i.e. the dry powder can directly be packaged and used. This among other things achieved by the fact that the particles of the dry powder have a mass

According to another aspect of the invention, the siRNA of the dry powder is active in silencing the translation of messenger RNA into proteins causing lung diseases. This enables very potent application opportunities, since the dry powder is, in some cases directly after the spray drying, is in a condition that can be used with a DPI. Thus, the treatment of lung diseases and the use of respective siRNA is a promising application of the inventive dry powder.

The above problem is also solved by a pharmaceutical product, in particular for the use with dry inhaler devices, comprising a packaging enclosing at least one sealed volume, preferably multiple sealed volumes, wherein the dry powder of any of the previously described embodiments is enclosed in the at least one sealed volume. This allows a very convenient use of the dry powder for a patient. The packaging can be built and shaped in such a form that in can be used with one or more different types of dry inhaler devices. In a preferred embodiment the packaging might be realized as a blister packaging. Preferably the packaging comprises multiple sealed volumes. According to a preferred embodiment, the packaging is constructed and shaped in such a way that it can be used in magazine function together with the dry inhaler device, allowing a sealed volume to be "loaded" into an application position and/or mechanically opened upon activating a preparation mechanism of the dry inhaler, such as a loading lever. Preferably the packaging is built to release the dry powder into a stream of gas, preferably air, upon activating a release mechanism of the dry inhaler device. In an alternative preferred embodiment the packaging may have the form and function of a capsule, in which the dry powder is enclosed.

### Firqures

Aspects of the invention are described by with respect to the figures, showing examples and advantageous embodiments of the invention.

Those figures show:
- Fig. 1:: A Scanning Electron Microscopy Image of an embodiment of the inventive dry powder;
- Fig. 2:: The results of an siRNA integrity test a the dry powder according to the invention.

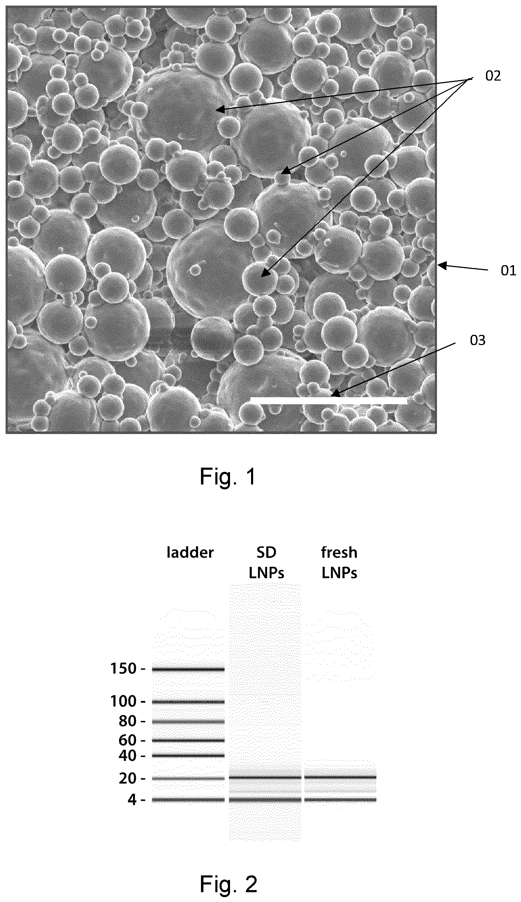

Fig. 1 shows a Scanning Electron Microscopy (SEM) image of the dry powder (01) according to the invention. The particles (02) of the dry powder show a spherical shape. As can be seen from the scale bar (03), which represent a length/distance of 10µm, that the particles (02) have a diameter of less than 10µm.

Fig. 2 shows the result of a siRNA integrity test (Bioanalyser). The second and the third column represent the comparison between fresh and spry dried lipid nanoparticles with the respective siRNA. The test bands/lines at the values 4 and 20 of the ladder are representative of structurally and functionally intact siRNA. It is shown in Fig. 2 that no internal lines between the ones of 4 and 20 are present for the dry powder. This means that no degradation of the siRNA has occurred and that the siRNA is intact in the dry powder.

## Claims

1. Nano-in-micro (NIM) encapsulated bioactive siRNA dry powder comprising lipid nanoparticles, the lipid nanoparticles comprising from at least an ionizable cationic lipid, a helper lipid, a stealth lipid, and siRNA,
wherein the lipid nanoparticles are encapsulated in sugar and/or sugar alcohol,
**characterized in that**
the mass ratio of siRNA to a sugar and/or sugar alcohol is more than 0.10%, especially between 0,90% and 1,30%.

2. Dry powder according to claim 1,
**characterized in that**
at least 75%, preferably at least 85% of the siRNA is bioactive in the dry powder state and/or after re-dispersion.

3. Dry powder according to claim 1 or 2,
**characterized in that**
residual moisture of the dry powder is less than 5%, in particular less than 3%.

4. Dry powder according to any of the previous claims,
**characterized in that** the lipid mass ratio of the dry powder is above 8%, preferably above 10%.

5. Dry powder according to any of the previous claims,
**characterized in that**
the sugar comprises or consists of lactose.

6. Dry powder according to any of the preceding claims,
**characterized in that**,
the lipid nano particles are according to an Onpattro^{®}-formulation or patisiran-formulation.

7. Dry powder according to claims 1 to 6,
used as a pharmaceutical dosage form, especially for pulmonary delivery.

8. Dry powder according to claim 1 to 7,
wherein the siRNA is active in silencing the translation of messenger RNA into proteins causing lung diseases.

9. Pharmaceutical product, in particular for the use with dry powder inhaler devices, comprising a packaging enclosing at least one sealed volume, preferably multiple sealed volumes, wherein a dry powder is enclosed in the sealed volume,
**characterized by**
a dry powder of any of the preceding claims.
